## Europäisches Patentamt
## European Patent Office
## Office européen des brevets

(11) Veröffentlichungsnummer: **0 155 344**
**B1**

(12) # EUROPÄISCHE PATENTSCHRIFT

(45) Veröffentlichungstag der Patentschrift:
14.12.88

(51) Int. Cl.⁴: **A 61 K 7/48**, A 61 K 33/00

(21) Anmeldenummer: **84108893.3**

(22) Anmeldetag: **27.07.84**

(54) **Hautpflegemittel.**

(30) Priorität: **02.08.83 DE 3327840**

(43) Veröffentlichungstag der Anmeldung:
**25.09.85 Patentblatt 85/39**

(45) Bekanntmachung des Hinweises auf die Patenterteilung:
**14.12.88 Patentblatt 88/50**

(84) Benannte Vertragsstaaten:
**AT BE CH DE FR GB IT LI LU NL SE**

(56) Entgegenhaltungen:
**WO-A-84/02652**
**FR-A-2 242 971**
**FR-A-2 242 991**
**FR-A-2 474 865**
**FR-A-2 489 689**
**FR-A-2 546 754**

**CHEMICAL ABSTRACTS, Band 82, Nr. 12, März 1975, Seite 261, Nr. 77002c, Columbus, Ohio, US; & JP - A - 74 81 547 (OHETSUCHI K.K.) 06.08.1974**

(73) Patentinhaber: **Blendax GmbH, Rheinallee 88, D-6500 Mainz (DE)**

(72) Erfinder: **Raaf, Helmut, Dr., Schönblick 7, D-6551 Bockenau (DE)**
Erfinder: **Bimczok, Rudolf, Dr., Am Keltenlager 49, D-6500 Mainz- Finthen (DE)**
Erfinder: **Ittel, Ingrid, Dr., Bahnhofstrasse 56, D-6507 Ingelheim (DE)**

# 0 155 344

**Beschreibung**

Die vorliegende Erfindung betrifft Hautpflegemittel mit ausgezeichneten hautpflegenden Eigenschaften. Von Hautpflegemitteln, insbesondere Hautcremes und Lotionen, erwartet der Verbraucher, daß sie sich auf der Haut gut verteilen lassen, rasch in diese einziehen, ohne einen störenden Fettfilm zu hinterlassen, und sich die Haut nach der Behandlung glatt und geschmeidig anfühlt.

Darüber hinaus soll ein Hautpflegemittel auch eine länger anhaltende Wirkung ausüben, d. h., die enthaltenden Wirkstoffe sollen in die Epidermis eindringen und auch eine Regeneration der Hornschichten bewirken.

Zur Lösung dieser Aufgabe wurden bereits zahlreiche Wirkstoffe vorgeschlagen, wobei insbesondere feuchtigkeitsregulierende Zusätze wie die Nachstellung des sogenannten NMF- (Natural Moisturizing Factor) Faktors eine wesentliche Rolle spielt.

Daneben sind auch Vitamine, Aminosäuren, Hydroxy- und Polycarbonsäuren, Polyalkohole, Harnstoff, die verschiedensten Pflanzenextrakte, durchblutungsfördernde und hyperämisierende Mittel, etc. zum Einsatz in Hautpflegemitteln zur Verbesserung des Zustandes der Haut vorgeschlagen worden. Es ist auch bereits bekannt, daß verschiedene Mineralstoffe für die Gesundheit der Haut eine wesentliche Rolle spielen. Diese Mineralstoffe werden benötigt als Baustoffe, als Co-Enzyme und Biokatalysatoren, zur Regulierung des Wasserhaushalts und des osmotischen Drucks, zur Erhaltung konstanter pH-Werte und bioelektrischer Potentiale.

Es wurde deshalb auch vorgeschlagen, solche Mineralstoffe alleine oder in Kombination systemisch zu verabreichen, um ein gesundes Erscheinungsbild der Haut aufrechtzuerhalten (vgl. beispielsweise M. Haas, Kosmetik-Journal vom 5. August 1982, S. 8 - 9).

Es wurde nun gefunden, daß durch eine genau qualitativ und quantitativ aufeinander abgestimmte Auswahl von verschiedenen Mineralsalzen bei deren Einsatz in Hautpflegemitteln auch eine ausgezeichnete lokale hautpflegende Wirkung erreicht werden kann.

Gegenstand der Erfindung ist daher ein Hautpflegemittel, das in an sich üblichen Grundlagen Verbindungen enthält, die ein Kationengemisch in folgenden Mengen (berechnet auf die jeweiligen Ionen) liefern:

Natrium: 0,01 - 5,0 Gew.-%, insbesondere 0,1 bis 2,5 Gew.-%;
Kalium: 0,01 - 3,0 Gew.-%, insbesondere 0,1 bis 1,5 Gew.-%;
Magnesium: 0,01 - 2,0 Gew.-%,insbesondere 0,1 bis 1,0 Gew.-%;
Calcium: 0,01 - 2,0 Gew.-%, insbesondere 0,1 bis 1,0 Gew.-%;
Zink: 0,01 - 1,0 Gew.-%, insbesondere 0,05 bis 0,5 Gew.-%, und
Kupfer: 0,0001-0,01 Gew.-%, insbesondere 0,0005 bis 0,005 Gew.-%,

berechnet auf die Gesamtzusammensetzung des Mittels.

Gemäß einer bevorzugten Ausführungsform enthält das erfindungsgemäß Hautpflegemittel zusätzlich weitere Mineralsalze, die folgende Zonen in den angegebenen Mengen liefern:

Mangan: 0,0001 - 0,01 Gew.-%, insbes. 0,001 bis 0,005 Gew.-%;
Eisen: 0,0001 - 0,01 Gew-%, insbes. 0,001 bis 0,005 Gew.-%;
Vanadium: 0,0001 - 0,01 Gew.-%, insbes. 0,001 bis 0,005 Gew.-%,
Aluminium: 0,0001 - 0,5 Gew.-%, insbes. 0,01 bis 0,1 Gew.-%; und/oder
Kobalt: 0,0001 - 0,01 Gew.-%, insbes. 0,001 bis 0,005 Gew.-%,

berechnet auf die Gesamtzusammensetzung des Mittels.

In den erfindungsgemäßen Hautpflegemitteln werden diese Ionen in Form ihrer geeigneten, physiologisch verträglichen Salze in entsprechenden Konzentrationen eingesetzt.

Solche Salze sind beispielsweise die Chloride, Sulfate, Phosphate, Citrate, Lactate, Glycerophosphate, Salze von Aminosäuren, Gluconate, Edetate, Tartrate, Malate, Mandelate, Benzoate, Salicylate, Phytate oder Cinnamate. Grundsätzlich ist jedes Salz der eingesetzten Elemente geeignet, das in der Lage ist, Ionen in den definierten Mengenbereichen in den erfindungsgemäßen Hautpflegemitteln abzugeben.

Zusätzlich zu den erfindungsgemäßen Mineralsalzmischungen können in den erfindungsgemäßen Hautpflegenitteln weitere, an sich bekannte Wirkstoffe anwesend sein.

Solche Wirkstoffe sind insbesondere die zur Hautpflege bereits eingesetzten Vitamine, wie die Vitamine A, $B_1$, $B_2$, $B_6$, $B_{12}$, D, E, K, H, Folsäure, Nicotinsäureamid und Pantothensäure bzw. D-Panthenol. Besonders bevorzugte Vitamine sind dabei Vitamin A, Vitamin E (Tocopherol) und D-Panthenol. Eine weitere, in den erfindungsgemäßen Hautpflegemitteln zum Einsatz geeignete, an sich bekannte Wirkstoffgruppe sind Aminosäuren wie Methionin, Cystin, Cystein, Alanin, Asparaginsäure, Leucin, Isoleucin, Lysin, Valin, Serin, Arginin, Glutaminsäure, Phenylalanin, Threonin, Tyrosin und Tryptophan. Geeignet sind auch niedermolekulare Eiweißhydrolysate.

Falls die erfindungsgemäßen Hautpflegemittel in Form von Sonnenschutzmitteln vorliegen, enthalten sie selbstverständlich die belannten UV-Strahlen absorbierenden Stoffe.

Writere geeignete, an sich bekannte Wirkstoffe sind die verschiedenen Pflanzenextrakte, durchblutungsfördernde und entzündungshemmende Stoffe, etc.

2

Generell ist festzustellen, daß in den erfindungsgemäßen Hautpflegemitteln zusätzlich zu den synergistisch wirkenden Mineralsalzmischungen alle physiologisch verträglichen, für die Hautpflege vorgeschlagenen Wirkstoffe mitverwendet werden können, vorausgesetzt selbstverständlich ihre Verträglichkeit mit den übrigen Bestandteilen der jeweiligen Rezeptur.

Die erfindungsgemäßen Hautpflegemittel können in jeder für Hautkosmetika geeigneten Applikationsform Einsatz finden. Bevorzugt sind hierbei Hautcremes, die in Form von Öl-in-Wasser-Emulsionen oder Wasser-in-Öl-Emulsionen unter Zusatz der bekannten Grund- und Hilfsstoffe verwendet werden können.

Es ist jedoch auch möglich, Lotionen, Gele oder Lösungen in Form von Haut- und Gesichtswässern als Grundlagen der erfindungsgemäßen Hautpflegemittel einzusetzen.

Diese Zusammensetzungen können selbstverständlich auch, unter Zusatz der üblichen Treibmittel, als Aerosole konfektioniert sein. Auch die Anwendung in Form von Gesichtspackungen bzw. Gesichtsmasken, Pasten oder Suspensionen ist möglich.

Die Zusammensetzung und Herstellung solcher Grundlagen sind dem Fachmann geläufig und in den verschiedenen einschlägigen Handbüchern ausführlich beschrieben, es wird hierzu, beispielhaft, auf K. Schrader, "Grundlagen und Rezepturen der Kosmetika" (1979, Dr. Alfred Hüthig Verlag, Heidelberg), insbesondere S. 227 bis 375, verwiesen.

Die erfindungsgemäßen Hautpflegemittel weisen vorzugsweise einen pH-Wert zwischen 4 und 8 auf; ein besonders geeigneter pH-Wert für die Anwendung auf der Haut liegt zwischen 4 und 6.

Im folgenden werden einige beispielhafte Rezepturen für erfindungsgemäß zusammengesetzte Hautpflegemittel gegeben.

Bei den angegebenen Prozentzahlen handelt es sich dabei jeweils um Gewichtsprozent.

**Beispiel 1**

Hautcreme für trockene Haut

| | |
|---|---|
| Siliconöl | 11,50 % |
| Kollagenhydrolysat | 0,50 % |
| Isopropylmyristat | 2,00 % |
| Glykoldistearat | 2,00 % |
| Weißöl | 10,00 % |
| Cetylalkohol | 3,50 % |
| Oleyloleat | 4,00 % |
| Glycerinmono-/polyoxiethylenstearat | 4,00 % |
| Fettalkoholethoxylat | 1,00 % |
| Mandelöl | 6,00 % |
| Glycerin | 4,00 % |
| Verdickungsmittel | 0,50 % |
| Konservierungsmittel | 0,55 % |
| Vitamin A (200,000 I.E.) | 0,05 % |
| Vitamin E | 0,05 % |
| D-Panthenol | 0,50 % |
| Parfum | 0,40 % |
| Natriumchlorid | 0,20 % |
| Kaliumchlorid | 0,10 % |
| Calciumchlorid. $2H_2O$ | 0,0375 % |
| Zinksulfat. $7H_2O$ | 0,025 % |
| Magnesiumsulfat. $7H_2O$ | 0,05 % |
| Kupfersulfat. $5H_2O$ | 0,0037 % |
| Ammoniumvandat | 0,0012 % |
| Mangansulfat. $H_2O$ | 0,00015 % |
| Entsalztes Wasser | ad 100 % |

pH-Wert: ca 4,5

**Beispiel 2**

Gesichtscreme für normale Haut

3

| | |
|---|---|
| Oleyloleat | 3,00 % |
| Siliconöl | 1,50 % |
| Weißöl | 5,00 % |
| Purcellinöl | 3,50 % |
| Cetylpamitat | 2,50 % |
| Stearylalkohol | 3,50 % |
| Glycoldistearat | 2,00 % |
| Sorbitanmonoleat | 2,00 % |
| Sorbitanpolyoxiethylenstearat | 2,00 % |
| Polyglykoläther | 1,00 % |
| Glycerin | 3,00 % |
| Parfum | 0,40 % |
| Konservierungsmittel | 0,35 % |
| Verdickungsmittel | 0,50 % |
| Vitamin A (200,000 I.E.) | 0,05 % |
| Vitamin E | 0,05 % |
| D-Panthenol | 0,50 % |
| Natriumchlorid | 0,25 % |
| Kaliumchlorid | 0,15 % |
| Calciumchlorid. $2H_2O$ | 0,04 % |
| Zinksulfat. $7H_2O$ | 0,02 % |
| Magnesiumsulfat. $7H_2O$ | 0,05 % |
| Kupfersulfat $5H_2O$ | 0,004 % |
| Mangansulfat. $H_2O$ | 0,0002 % |
| Entsalztes Wasser | ad 100 % |

pH-Wert: ca. 4,5

**Beispiel 3**

Handlotion

| | |
|---|---|
| Glycerinmonostearat | 4,00 % |
| 2-Ethylhexylpalmitat | 17,00 % |
| Sorbit (60 %) | 4,00 % |
| Siliconöl | 0,75 % |
| Purcellinöl | 3,00 % |
| Sorbitanpolyoxiethylenstearat | 2,00 % |
| Parfum | 0,45 % |
| Polyglykoläther | 1,20 % |
| Aminosäuregemisch | 0,80 % |
| Natriumchlorid | 0,25 % |
| Kaliumchlorid | 0,10 % |
| Magnesiumchlorid | 0,10 % |
| Calciumchlorid. $2H_2O$ | 0,05 % |
| Zinkchlorid | 0,03 % |
| Kupferchlorid | 0,005 % |
| Eisen (II) sulfat. $2H_2O$ | 0,001 % |
| Aluminiumsulfat | 0,05 % |
| Konservierungsmittel | 0,30 % |
| Wasser | ad 100 % |

**Beispiel 4**

Feuchtigkeitsmilch

4

| | |
|---|---|
| Polyoxiethylenfettsäureester | 5,50 % |
| Glycerinsorbitanfettsäureester | 2,50 % |
| Isopropylmyristat | 4,00 % |
| Decyloleat | 4,00 % |
| Fettsäureester-Gemisch (PCα-liquid) (PCL-liguid) | 4,00 % |
| 2-Octyldodecanol | 2,00 % |
| Perhydrosqualen | 8,00 % |
| Glycerin | 2,00 % |
| 1,2-Propylenglykol | 1,80 % |
| Harnstoff | 2,00 % |
| Aminosäurekomplex | 0,50 % |
| Parfum | 0,40 % |
| Konservierungsmittel | 0,25 % |
| Natriumlactat | 1,00 % |
| Magnesiumsulfat. $7H_2O$ | 0,80 % |
| Kaliumcitrat. $H_2O$ | 0,90 % |
| Zinkcitrat. $2H_2O$ | 0,35 % |
| Calciumgluconat. $H_2O$ | 0,50 % |
| Kupfersalicylat. $4H_2O$ | 0,005 % |
| Wasser | ad 100 % |

**Beispiel 5**

Sonnenschutzcreme

| | |
|---|---|
| Cetylalkohol | 17,50 % |
| Polyoxiethylensorbitanlanolat | 4,00 % |
| Sorbitanmonolaurat | 2,50 % |
| Polyoxiethylensorbitanmonolaurat | 6,00 % |
| 2-Ethoxyethyl-p-methoxycinnamat | 2,50 % |
| Glycerin | 3,50 % |
| Konservierungsmittel | 0,25 % |
| Parfum | 0,50 % |
| Natriumchlorid | 0,50 % |
| Kaliumchlorid | 0,60 % |
| Magnesiumsulfat. $7H_2O$ | 0,70 % |
| Zinkcitrat. $2H_2O$ | 0,20 % |
| Calciumchlorid. $2H_2O$ | 0,20 % |
| Kupfersulfat. $5H_2O$ | 0,005 % |
| Cobalt (II) chlorid. $2H_2O$ | 0,0005 % |
| Aluminiumchlorid. $6H_2O$ | 0,02 % |
| Manganchlorid. $4H_2O$ | 0,003 % |
| Eisen (III) chlorid. $6H_2O$ | 0,005 % |
| Wasser | ad 100 % |

**Beispiel 6**

Körperlotion

5

| | |
|---|---|
| Polyoxyethylene oleyl ether (2EO-Gruppen) | 2,50 % |
| Polyoxyethylene oleyl ether (20 EO-Gruppen) | 4,50 % |
| Lanolinalkohole (Lipocol ®) | 3,00 % |
| Bienenwachs | 5,00 % |
| Isopropylisostearat | 12,00 % |
| Cetylalkohol | 6,00 % |
| Siliconöl | 0,30 % |
| Glycerin | 3,50 % |
| Konservierungsmittel | 0,35 % |
| Parfum | 0,40 % |
| Eiweißhydrolysat | 0,60 % |
| Natriumdihydrogenphosphat. $2H_2O$ | 1,00 % |
| Kaliummonohydrogenphosphat | 0,75 % |
| Calciumglycerophosphat | 0,80 % |
| Magnesiumcitrat. $5H_2O$ | 0,65 % |
| Zinklactat. $2H_2O$ | 0,20 % |
| Kupferlactat. $2H_2O$ | 0,01 % |
| Mangannitrat. $4H_2O$ | 0,001 % |
| D-Panthenol | 0,10 % |
| Vitamin $B_6$ (Hydrochlorid) | 0,10 % |
| Vitamin E (Tocopherolacetat) | 0,10 % |
| Vitamin F | 0,25 % |
| Wasser | ad 100 % |

**Beispiel 7**

<u>Gesichtswasser</u>

| | |
|---|---|
| Ethanol | 8,00 % |
| 1-Methoxypropanol-2 | 7,00 % |
| Hamamelisextrakt | 0,50 % |
| Ester hydrierter Ricinusölfettsäure mit oxethyliertem Glycerin | 0,30 % |
| D-Panthenol | 0,50 % |
| Milchsäure | 0,50 % |
| Aluminiumlactat | 0,50 % |
| Natriumlactat | 1,20 % |
| Kaliumbenzoat. $3H_2O$ | 0,50 % |
| Zinkchlorid | 0,25 % |
| Magnesiumlactat. $3H_2O$ | 0,30 % |
| Calciumchlorid. $2H_2O$ | 0,60 % |
| Kupfersulfat. $5H_2O$ | 0,005 % |
| Manganchlorid. $4H_2O$ | 0,005 % |
| Eisen (III) chlorid | 0,005 % |
| Parfum | 0,15 % |
| Wasser | ad 100 % |

**Beispiel 8**

<u>Hautbehandlungs- und -schutzgel</u>

| | |
|---|---|
| Natriumalginat | 1,80 % |
| Sorbit, 60-%-ig | 5,00 % |
| DL-Serin | 0,50 % |
| Aminosäurekomplex | 1,00 % |
| Vitamin A, wasserlöslich | 0,50 % |
| Harnstoff | 2,00 % |
| Polyvinylpyrrolidon | 2,00 % |
| Glucose | 1,00 % |
| Konservierungsmittel | 0,20 % |
| Parfumöl | 0,20 % |
| Natriumchlorid | 1,00 % |
| Kaliumchlorid | 1,00 % |
| Magnesiumchlorid. 6H$_2$O | 0,70 % |
| Calciumchlorid. 2H$_2$O | 0,85 % |
| Zinksulfat. 7H$_2$O | 0,20 % |
| Kupfersulfat. 5H$_2$O | 0,01 % |
| Mangansulfat. H$_2$O | 0,005 % |
| Natriummetavanadat | 0,005 % |
| Wasser | ad 100 % |

**Beispiel 9**

Gesichtsschaummaske

| | |
|---|---|
| Cetylstearylalkohol | 2,80 % |
| Cetylstearylsulfat, Natriumsalz | 4,00 % |
| Gemisch aus Mono-, Di- und Tri(alkyl tetraglykolether) orthophosphorsäureestern (Hostaphat$^R$ KW 340) | 2,80 % |
| Ethanol | 3,50 % |
| Sorbitlösung, 60-%-ig | 2,00 % |
| Isopropylmyristat | 3,20 % |
| Allantoin | 0,10 % |
| Konservierungsmittel | 0,20 % |
| Parfum | 0,20 % |
| Natriumlactat | 1,20 % |
| Kaliumvanadiumsulfat. 12H$_2$O | 0,02 % |
| Kaliummolybdat | 0,003 % |
| Kaliumglycerophosphat | 0,50 % |
| Calciumlactat. 5H$_2$O | 0,55 % |
| Aluminiumlactat | 0,35 % |
| Magnesiumlactat. 3H$_2$O | 0,40 % |
| Kupferchlorid | 0,001 % |
| Wasser | ad 100 % |

90 Gew.-% dieser Zusammensetzung werden mit 10 Gew.-% eines Treibmittelgemisches in Aerosoldosen abgefüllt.

**Patentansprüche**

1. Hautpflegemittel auf Basis üblicher Grund- und Zusatzstoffe, gekennzeichnet durch einen Gehalt an folgenden Ionen liefernden Verbindungen in folgenden Konzentrationen, wobei sich die angegebenen Mengen auf die jeweiligen Kationen beziehen:

Natrium: 0,01 - 5,0 Gew.-%, insbesondere 0,1 bis 2,5 Gew.-%;
Kalium: 0,01 - 3,0 Gew.-%, insbesondere 0,1 bis 1,5 Gew.-%;
Magnesium: 0,01 - 2,0 Gew.-%, insbesondere 0,1 bis 1,0 Gew.-%;
Calcium: 0,01 - 2,0 Gew.-%, insbesondere 0,1 bis 1,0 Gew.-%;
Zink: 0,01 - 1,0 Gew.-%, insbesondere 0,05 bis 0,5 Gew.-% und
Kupfer; 0,0001 - 0,01 Gew.-%, insbesondere 0,0005 bis 0,005 Gew.-%,

berechnet auf die Gesamtzusammensetzung des Mittels.

2. Hautpflegemittel nach Anspruch 1, gekennzeichnet durch einen Gehalt an mindestens einer weiteren, folgende Ionen liefernden Verbindung, wobei sich die angegebenen Mengen auf die Ionen beziehen:

Mangan:      0,0001 - 0,01 Gew.-%, insbes. 0,001 bis 0,005 Gew.-%;
Eisen:       0,0001 - 0,01 Gew.-%, insbes. 0,001 bis 0,005 Gew.-%;
Vanadium:    0,0001 - 0,01 Gew.-%, insbes. 0,001 bis 0,005 Gew.-%;
Aluminium:   0,001 - 0,5 Gew.-%, insbes. 0,01 bis 0,1 Gew.-%; und/oder
Kobalt:      0,0001 - 0,01 Gew.-%, insbes. 0,001 bis 0,005 Gew.-%,

berechnet auf die Gesamtzusammensetzung des Mittels.

3. Hautpflegemittel nach Anspruch 1 und/oder 2, gekennzeichnet durch einen zusätzlichen Gehalt an Vitaminen.

4. Hautpflegemittel nach Anspruch 4, gekennzeichnet durch einen Gehalt an Vitamin A, Vitamin E und/oder D-Panthenol.

5. Hautpflegemittel nach einem der vorhergehenden Ansprüche, dadurch gekennzeichnet, daß sein pH-Wert zwischen 4 und 8 beträgt.

6. Hautpflegemittel nach Anspruch 5, dadurch gekennzeichnet, daß sein pH-Wert zwischen 4 und 6 liegt.

## Claims

1. Skin-care agent on the basis of the usual components and additives, characterized by a content of compounds releasing the following ions in the following concentrations, related to the corresponding cations:

sodium:      0,01 - 5,0 % by weight, particularly 0,1 - 2,5 % by weight;
potassium:   0,01 - 3,0 % by weight, particularly 0,1 - 1,5 % by weight;
magnesium:   0,01 - 2,0 % by weight, particularly 0,1 - 1,0 % by weight;
calcium:     0,01 - 2,0 % by weight, particularly 0,1 - 1,0 % by weight;
zinc:        0,01 - 1,0 % by weight, particularly 0,05 - 0,5 % by weight;
copper:      0,0001 - 0,01 % by weight, particularly 0,0005 to 0,005 % by weight,

all percentages calculated to the total composition.

2. Skin-care agent according to claim 1, characterized by a content of at least one more compound supplying the following ions in the indicated amounts, referred to the ions:

manganese:   0,0001 - 0,01 % by weight, particularly 0,001 - 0,005 % by weight;
iron:        0,0001 - 0,01 % by weight, particularly 0,001 - 0,005 % by weight;
vanadium:    0,0001 - 0,01 % by weight, particularly 0,001 - 0,005 % by weight;
aluminum:    0,001 - 0,5 % by weight; particularly 0,01 - 0,1 % by weight; and/or
cobalt:      0,0001 - 0,01 % by weight; particularly 0,001 - 0,005 % by weight;

calculated to the total composition.

3. Skin-care agent according to claim 1 and/or 2, characterized by an additional content of vitamins.

4. Skin-care agent according to claim 3, characterized by a content of vitamin A, vitamin E and/or D-panthenol.

5. Skin-care agent according to one of the preceding claims, characterized by the fact that its pH-value is between 4 and 8.

6. Skin-care agent according to claim 5, characterized by the fact that its pH-value is between 4 and 6.

## Revendications

1. Produit pour les soins de la peau à base d'une substance de base et d'additifs, caractérisé par une teneur en composés fournissant les ions suivants aux concentrations suivantes, les proportions indiquées se rapportant aux cations respectifs:

Sodium:      0,01 - 5,0 % en poids, en particulier 0,1 à 2,5 % en poids,
Potassium:   0,01 - 3,0 % en poids, en particulier 0,1 à 1,5 % en poids,
Magnésium:   0,01 - 2,0 % en poids, en particulier 0,1 à 1,0 % en poids,
Calcium:     0,01 - 2,0 % en poids, en particulier 0,1 à 1,0 % en poids,
Zinc:        0,01 - 1,0 % en poids, en particulier 0,05 à 0,5 % en poids, et
Cuivre:      0,0001 - 0,01 % en poids, en particulier 0,0005 à 0,005 % en poids,

et étant calculées par rapport à la composition globale du produit.

2. Produit pour les soins de la peau suivant la revendicaton 1, caractérisé par une teneur en au moins un autre composé fournissant les ions suivants, proportions indiquées se rapportant aux ions:

Manganèse: 0,0001 - 0,01 % en poids, en particulier 0,001 à 0,005 % en poids,
Fer: 0,0001 - 0,01 % en poids, en particulier 0,001 à 0,005 % en poids,
Vanadium: 0,0001 - 0,01 % en poids, en particulier 0,001 à 0,005 % en poids,
Aluminium: 0,001 - 0,5 % en poids, en particulier 0,01 à 0,1 % en poids, et/ou
Cobalt: 0,0001 - 0,01 % en poids, en particulier 0,001 à 0,005 % en poids,

et étant calculées par rapport à la composition globale du produit.

3. Produit pour les soins de la peau suivant l'une ou l'autre des revendications 1 et 2, caractérisé par une teneur supplémentaire en vitamines.

4. Produit pour les soins de la peau suivant la revendication 3, caractérisé par une teneur en vitamine A, en vitamine E et/ou en D-panthénol.

5. Produit pour les soins de la peau suivant l'une des revendications précédentes, caractérisé en ce que sa valeur de pH est comprise entre 4 et 8.

6. Produit pour les soins de la peau suivant la revendication 5, caractérisé en ce que sa valeur de pH est comprise entre 4 et 6.